# FASCICULE DE BREVET EUROPEEN

(11) **EP 4 444 373 B1**
(45) Date de publication et mention de la délivrance du brevet: **03.12.2025**
(21) Numéro de dépôt: 22802674.6
(22) Date de dépôt: 21.10.2022
(51) Int. Cl.: A61M 1/00, A61K 35/35, C12M 1/00

(54) **DISPOSITIF DE PURIFICATION DE TISSU ADIPEUX**
VORRICHTUNG ZUR REINIGUNG VON FETTGEWEBE
DEVICE FOR PURIFYING ADIPOSE TISSUE

(30) Priorité: 08.12.2021 FR 2113135
(43) Date de publication de la demande: 16.10.2024
(73) Titulaire: Neosyad, 13290 Aix-en-Provence (FR)
(72) Inventeur: ROCHE, Régis, 83510 Lorgues (FR); CABAUD, François, 13510 Eguilles (FR); NELISSEN, Xavier, 4453 Villers Saint Simeon (BE)
(74) Mandataire: Cabinet Beau de Loménie
(86) Numéro de dépôt international: PCT/FR2022/052006
(87) Numéro de publication internationale: WO 2023/105126

(56) Documents cités:
- US-A1- 2019 322 979
- US-A1- 2020 061 259

## Description

### Domaine Technique

La présente invention concerne un dispositif de purification de tissu adipeux. L'invention trouve notamment une application pour les opérations de transplantation de graisse (désignées par « lipofilling » en langue anglaise) à visées esthétiques et reconstructrices. L'invention trouve, en particulier, une utilisation pour la transplantation de graisse utile pour les opérations de chirurgie mammaire, la mise en œuvre du dispositif objet de l'invention n'étant toutefois pas limitée à cette application. Un exemple de dispositif de l'art antérieur est décrit dans US 2020/061259 A1.

### Technique antérieure

Des opérations de transplantation de graisse ou de greffe autologue de tissu adipeux sont utilisées en chirurgie notamment en chirurgie du fessier pour une augmentation de volume ou en chirurgie mammaire afin de modeler le sein pour lui donner un aspect plus naturel après un DIEP (pour « Deep Inferior Epigastric Perforator » ou Perforante de l'artère épigastrique profonde intérieure), une reconstruction par lambeau de grand dorsal, ou après le placement d'un implant mammaire. Ces opérations consistent à prélever de la graisse d'un patient dans une zone donneuse pour la réintroduire ensuite dans la zone d'intérêt du corps du patient.

Cependant, les techniques connues de transplantation de graisse présentent un inconvénient important, à savoir un taux important de résorption de la graisse implantée. En effet, il est généralement constaté une résorption d'au moins 50% de la graisse implantée (perte de volume) six mois après l'opération de transplantation. Il est, par conséquent, souvent nécessaire de faire plusieurs interventions sur le patient pour obtenir un résultat final satisfaisant. Ces interventions répétées ne sont pas souhaitables tant pour le confort du patient que pour le coût et la durée du traitement.

Le tissu adipeux prélevé contient un milieu liquide constitué principalement, d'une part, d'huile et de sang présents initialement dans le tissu adipeux et, d'autre part, d'un liquide tel qu'une solution physiologique introduite lors du prélèvement du tissu adipeux et/ou lors d'une opération de lavage de celui-ci.

Les inventeurs ont déterminé que le taux de résorption est en grande partie lié à la quantité de liquide présente dans le tissu adipeux réintroduit dans le corps du patient. En d'autres termes, plus il reste de liquide dans le tissu adipeux réinjecté, plus le taux de résorption est important.

Il est donc important de traiter le tissu adipeux avant sa réintroduction dans le corps du patient afin de retirer un maximum de milieu liquide.

Le document US 2020/0054824 divulgue un système de transplantation de graisse qui utilise un dispositif de filtration par gravité commercialisé sous la référence commerciale Revolve^{®}.

Cependant, ce type de dispositif de filtration ne permet pas de retirer du tissu adipeux une quantité suffisante de liquide apte à diminuer le taux de résorption de manière significative.

Une autre solution connue consiste à centrifuger le tissu adipeux afin de séparer les phases par densité. Cette solution ne permet pas non plus de retirer du tissu adipeux une quantité optimale de liquide. En outre, pour que la séparation des phases soit efficace, la centrifugation doit se faire à des accélérations centrifuges élevées, généralement supérieures ou égales à 400G, pouvant même très souvent atteindre 900G. Ces vitesses/accélérations sont délétères pour le tissu, et génèrent une mort cellulaire comprise classiquement entre 10% et 40% selon les accélérations réalisées.

### Exposé de l'invention

L'invention est définie par les revendications annexées.

La présente invention vise à surmonter les inconvénients de l'art antérieur en proposant un dispositif pour la purification d'un tissu adipeux comprenant au moins une enceinte étanche, un filtre présent dans l'enceinte étanche, ledit filtre délimitant une chambre de centrifugation pour un tissu adipeux et un moyen d'entrainement en rotation du filtre, le filtre présentant une taille de pore configurée pour laisser passer un milieu liquide et retenir un tissu adipeux.

Le dispositif de purification de l'invention combine ainsi avantageusement l'utilisation d'un filtre apte à drainer un milieu liquide d'un tissu adipeux avec la mise en rotation de celui-ci. En effet, le filtre formant une chambre de centrifugation, un tissu adipeux présent à l'intérieur de cette chambre peut être soumis à des accélérations centrifuges contre la paroi interne du filtre. Le milieu liquide présent dans et autour du tissu adipeux est alors efficacement drainé en dehors de la chambre de centrifugation à travers les pores du filtre alors que le tissu adipeux est retenu dans la chambre. On élimine ainsi une quantité importante de liquide permettant de disposer d'un tissu adipeux purifié qui, une fois réimplanté, présente un taux de résorption bien inférieur à celui obtenu avec les solutions de filtration de l'art antérieur.

Le dispositif de purification de l'invention présente en outre l'avantage de pouvoir drainer une quantité importante de liquide du tissu adipeux, et ce sans abimer celui-ci. En effet, les solutions de l'art antérieur qui utilisent la centrifugation afin de séparer les phases par densité et éliminer le liquide l'art antérieur sont mises en œuvre dans des enceintes closes comme des tubes ou des seringues. Pour que la séparation des phases soit efficace, la centrifugation doit se faire à des accélérations centrifuges élevées, généralement supérieures ou égales à 400G, pouvant même très souvent atteindre 900G. Ces vitesses/accélérations sont délétères pour le tissu adipeux, et génèrent une mort cellulaire comprise classiquement entre 10 et 40% selon les accélérations réalisées.

Dans le dispositif de purification de l'invention, la chambre de centrifugation n'est pas close puisque sa paroi est formée d'un filtre. L'évacuation des liquides n'est plus réalisée par séparation de phase, mais par un passage à travers les pores du filtre. Il n'est donc pas nécessaire de faire tourner rapidement le dispositif pour évacuer les liquides. Une accélération généralement comprise entre 5G et 25G est suffisante. De fait, le tissu adipeux ne subit que très peu de dommages. Par ailleurs, l'huile éventuellement générée (marqueur de la mort cellulaire et qu'il faut éviter de réinjecter car cela peut former des kystes huileux) est avantageusement éliminée également grâce au filtre, ce qui n'est pas le cas lors d'une centrifugation par séparation de phase, où l'huile se trouve au-dessus du tissu.

Le dispositif de purification comprend en outre un plateau de collecte présent dans la chambre de centrifugation, le plateau de collecte étant mobile en translation suivant l'axe de rotation du filtre. Le plateau de collecte joue un rôle de piston qui permet de racler la paroi interne du filtre de manière à collecter un maximum de tissu adipeux et de faciliter le prélèvement du tissu adipeux purifié en sommet du dispositif.

Selon un aspect particulier, le dispositif de purification comprend en outre une tige filetée s'étendant dans la chambre de centrifugation et coopérant avec une portion taraudée du plateau de collecte, la tige filetée étant reliée au moyen d'entrainement en rotation du filtre, ledit moyen étant configuré pour entraîner la tige filetée en rotation suivant un sens de rotation opposé au sens de rotation du filtre. On utilise ici avantageusement le même moyen d'entrainement en rotation à la fois pour la mise en centrifugation du filtre et le déplacement en translation verticale du plateau de collecte.

Selon un autre aspect particulier, le dispositif de purification comprend en outre une gaine de protection entourant la tige filetée. Cette gaine permet d'éviter à la tige filetée d'entrer en contact avec le tissu adipeux qui en s'accumulant au niveau du taraudage du plateau peut bloquer le déplacement de celui-ci.

Dans un exemple de réalisation, le dispositif de purification comprend en outre un élément de rigidification présent entre l'enceinte étanche et le filtre.

Dans un autre exemple de réalisation, le filtre est en un matériau rigide autoporteur. Dans ce cas, un élément de rigidification n'est plus nécessaire dans le dispositif de purification.

Le moyen d'entrainement en rotation du filtre peut être motorisé, par exemple avec un moteur électrique ou être manuel.

### Brève description des dessins

[Fig. 1] La figure 1 est une vue schématique éclatée d'un dispositif de purification conformément à un exemple de réalisation de l'invention,
[Fig. 2] La figure 2 est une vue schématique en coupe du dispositif de purification de la figure 1 une fois assemblé,
[Fig. 3] La figure 3 est une vue schématique en coupe montrant le dispositif de purification de la figure 2 après déplacement du plateau de collecte.

### Description des modes de réalisation

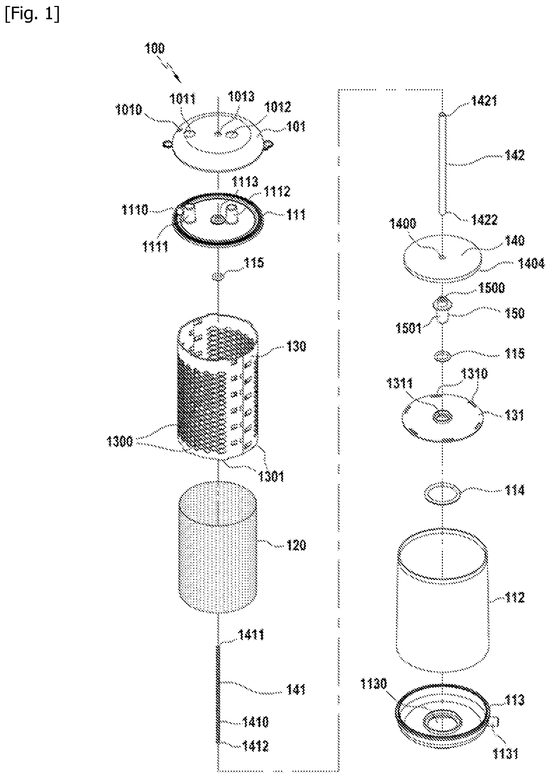

Les figures 1 à 3 illustrent un dispositif de purification 100 conformément à un mode de réalisation de l'invention.

Le dispositif de purification 100 comprend une enceinte étanche 110 formée ici par un capot 111, une paroi cylindrique 112 et un fond 113. Ces éléments sont fixés ensemble de manière étanche.

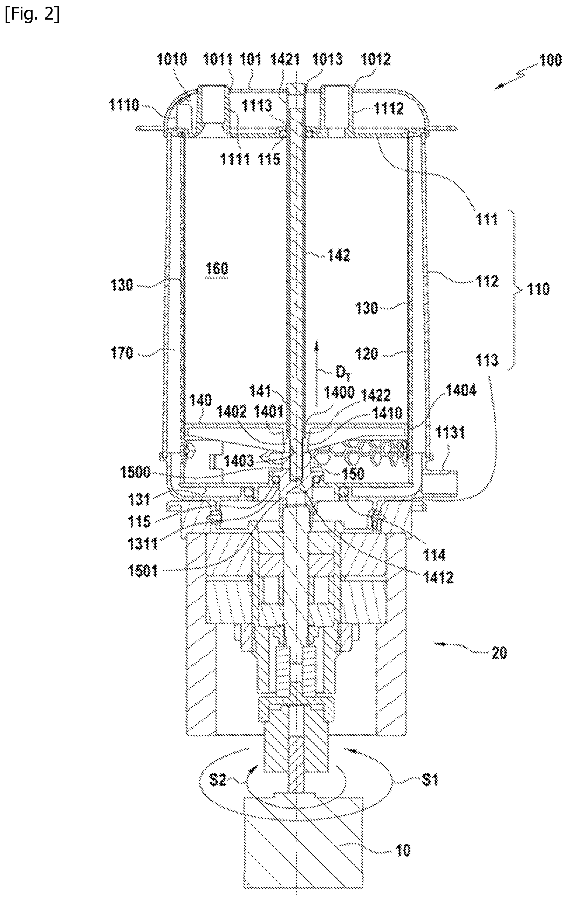

Le dispositif de purification 100 comprend également un filtre ou crible 120 présent dans l'enceinte étanche 110. Le filtre 120 délimite une chambre de centrifugation 160 (figure 2) dont le fonctionnement est décrit plus loin. Dans l'exemple décrit ici le filtre 120 présente une forme cylindrique. Le filtre peut présenter d'autres formes adaptées pour la centrifugation. Le filtre 120 présente une taille de pore configurée pour laisser passer un milieu liquide et retenir un tissu adipeux. La taille de pore est choisie en particulier pour permettre le passage de liquides tels que de l'huile, du sang, de l'eau ou une solution physiologique tout en retenant le tissu adipeux. A titre d'exemples non limitatifs, la taille de pore du filtre peut être comprise entre 50 µm et 1500 µm et plus préférentiellement entre 200 µm et 500 µm. Le filtre peut être réalisé de différentes manières comme par exemple par tressage, soudage de fils de plastique ou à partir d'un matériau comprenant des ajours ou trous ayant des dimensions correspondant à la taille de pore souhaitée. Le filtre peut être notamment en un matériau polymérique tel que du polyester ou du polypropylène mais l'homme du métier reconnaîtra que d'autres matériaux peuvent être utilisés. Dans l'exemple décrit ici, un élément de rigidification 130 de forme cylindrique est présent entre la paroi cylindrique 112 de l'enceinte étanche 110 et le filtre 120. Il a notamment pour fonction d'assurer la tenue structurale du filtre 120 pendant la centrifugation. L'élément de rigidification 130 est par exemple réalisé en matériau métallique ou plastique et présente une structure ajourée définissant une pluralité d'ouvertures 1300 afin de permettre l'évacuation du milieu liquide drainé par le filtre 120.

L'élément de rigidification 130 est associé à un plateau rotatif 131. Plus précisément, l'élément de rigidification 130 comporte à son extrémité inférieure des dents 1301 qui coopèrent avec des rainures 1310 présente au voisinage de la périphérie externe du plateau rotatif 131. Bien entendu, tout autre moyen de solidarisation de l'élément de rigidification avec le plateau rotatif, comme par exemple un clipsage ou collage, peut être envisagé. Le plateau rotatif 131 est relié à un moyen d'entrainement en rotation qui peut être manuel ou motorisé. Dans l'exemple décrit ici, le plateau rotatif est relié à un moteur électrique 10 via un embrayage bidirectionnel 20 configuré pour assurer l'entrainement en rotation du plateau rotatif 131 suivant un premier sens de rotation S₁ du moteur électrique 10 (figure 2). Le moteur électrique 10 peut être par exemple un moteur pas à pas ou un moteur à courant continu sans balai. L'embrayage bidirectionnel peut être remplacé par tout dispositif permettant un entraînement en rotation sélectif suivant deux sens de rotation opposés. Des joints 114 et 115 sont placés respectivement en-dessous et au-dessus du plateau rotatif 131 afin d'assurer l'étanchéité dans la partie inférieure du dispositif de purification.

La centrifugation est réalisée par mise en rotation du filtre 120. Plus précisément, le moteur électrique 10 est commandé suivant un premier sens de rotation S₁ pour entrainer le plateau rotatif 131 et l'élément de rigidification 130 en prise avec le plateau 131. La mise en rotation du plateau 131 et de l'élément de rigidification 130 entraine la mise en rotation du filtre 120. Le filtre est fixé à l'élément de rigidification par tout type de moyen adapté. A titre d'exemples non limitatifs, le filtre peut être fixé avec un ou plusieurs des moyens suivants : collage, engagement dans des crans de calage présents sur l'élément de rigidification, clipsage. La vitesse du moteur électrique 10 est contrôlée de manière à appliquer au tissu adipeux présent dans la chambre de centrifugation une accélération centrifuge. Ainsi, un tissu adipeux présent dans la chambre de centrifugation 160 sera soumis à une force centrifuge contre la paroi interne du filtre 120, ce qui permet de drainer efficacement le milieu liquide présent dans le tissu adipeux sans abimer celui-ci.

La centrifugation mise en œuvre peut être réalisée en imposant au filtre 120 une accélération supérieure ou égale à 8 G, par exemple supérieure ou égale à 10 G ou supérieure ou égale à 12G durant tout ou partie de la centrifugation. Cette accélération mesurée en G correspond au rapport entre l'accélération subie par le matériau et l'accélération de la pesanteur terrestre, laquelle est d'environ 9,81 m²/s. L'accélération subie par le matériau correspond au rapport de la force centrifuge appliquée et de la masse du matériau considéré. La force centrifuge appliquée est égale à m*ω² * R où m est la masse de l'objet considéré, ω est la vitesse angulaire du filtre 120 exprimée en rad/s et R la distance de l'axe X de rotation au centre de gravité de l'objet considéré.

Durant une phase de centrifugation, le tissu adipeux et les matières polluantes peuvent subir une accélération inférieure ou égale à 40 G, par exemple inférieure ou égale à 30 G, voire inférieure ou égale à 25 G ou inférieure ou égale à 20 G. Cette accélération peut être comprise entre 8 G et 40 G ou entre 8 G et 30 G ou entre 8 G et 25 G ou entre 8 G et 20 G. Cette accélération peut être comprise entre 10 G et 40 G ou entre 10 G et 30 G ou entre 10 G et 25 G ou entre 10 G et 20 G. Cette accélération peut être comprise entre 12 G et 40 G ou entre 12 G et 30 G ou entre 12 G et 25 G ou entre 12 G et 20 G. Les valeurs d'accélération décrites plus haut sont suffisantes pour permettre une élimination très significative des liquides présents dans le tissu adipeux et notamment de l'eau interstitielle et améliorer davantage encore la qualité du tissu adipeux purifié. Ce dernier point est plus difficile à réaliser dans le cas d'une filtration par gravité comme décrite dans US 2020/0054824. Par ailleurs, le fait de limiter l'accélération à des valeurs bien inférieures à celles mises en œuvre en centrifugation classique comme décrit ci-dessus, participe également à obtenir un tissu adipeux de qualité optimale et avec une légère quantité d'eau interstitielle résiduelle lui permettant d'être facilement injecté dans le corps du patient.

La centrifugation peut avoir une durée supérieure ou égale à 10 secondes, par exemple comprise entre 10 secondes et 60 secondes, préférentiellement entre 15 et 45 secondes.

Durant la centrifugation, le milieu liquide traversant le filtre 120 et l'élément de rigidification 130 est collecté dans un volume 170 délimité entre l'élément de rigidification 130 et la paroi cylindrique 112 de l'enceinte 110. Le liquide est ensuite évacué via un port d'évacuation 1131 présent sur le fond 113 de l'enceinte 110. Dans l'exemple décrit ici, le capot 111 comprend trois ports 1110, 1111 et 1112 destinés à être reliés respectivement à un dispositif d'aspiration de tissu adipeux prélevé depuis le corps d'un patient, à un dispositif de délivrance de liquide de lavage par exemple une solution physiologique et un dispositif de réimplantation ou de réintroduction de tissu adipeux. Le dispositif de purification comprend un couvercle 101 comportant des ouvertures 1010, 1011 et 1012 qui coopèrent avec les ports 1110, 1111 et 1112 du capot 111.

De manière optionnelle, le dispositif de purification peut en outre comprendre un plateau de collecte mobile à l'intérieur de la chambre de centrifugation. Comme illustré dans l'exemple des figures 1 et 2, le dispositif de purification 100 comprend en outre un plateau de collecte 140 qui est mobile en translation dans une direction D_{T} suivant l'axe X du filtre 120. Plus précisément, le dispositif de purification 100 comprend une tige filetée 141 qui s'étend verticalement à l'intérieur de la chambre de centrifugation 160 suivant l'axe X du filtre 120. L'extrémité inférieure 1412 de la tige filetée est reliée au moteur électrique 10 par l'intermédiaire d'un guide 150 et de l'embrayage bidirectionnel 20. Le guide 150 comprend un logement 1500 dans lequel l'extrémité inférieure 1412 est fixée. Une portion inférieure 1501 du guide 150 est connectée à une partie de l'embrayage bidirectionnel 20 qui est en prise avec le moteur électrique 10 que lorsque celui-ci transmet un mouvement de rotation suivant un deuxième sens de rotation S₂ opposé au premier sens de rotation S₁ utilisé pour la centrifugation. Lorsque le moteur électrique 10 tourne suivant le premier sens de rotation S₁, aucun mouvement de rotation n'est transmis par l'embrayage bidirectionnel 20 au guide 150 auquel est relié la tige filetée 141. De même, lorsque le moteur 10 tourne suivant le deuxième sens de rotation S₂, aucun mouvement de rotation n'est transmis par l'embrayage bidirectionnel 20 au plateau rotatif 131 et, par conséquent, au filtre 120. Le plateau de collecte 140 comporte sur sa face supérieure une ouverture centrale 1400 prolongée par un col 1401 qui s'étend à partir de la face inférieure du plateau 140. Le col 1401 comprend une portion 1402 comportant un taraudage 1403 qui coopère avec un filetage 1411 de la tige filetée 141. Ainsi, lorsque que la tige filetée est entrainée en rotation suivant le deuxième sens de rotation S₂, le plateau de collecte 140 s'élève dans la chambre de centrifugation 160 suivant la direction D_{T}.

Le bord périphérique 1404 du plateau de collecte 140 est en vis-à-vis de la paroi interne du filtre 120. Ainsi, lorsque le plateau de collecte 140 est déplacé en translation verticale suivant la direction D_{T}, celui-ci joue un rôle de piston qui permet de racler la paroi interne du filtre de manière à collecter un maximum de tissu adipeux. La translation verticale du plateau 140 permet également de placer le tissu adipeux collecté au plus près du capot 111 et de faciliter, par conséquent, son prélèvement pour réimplantation.

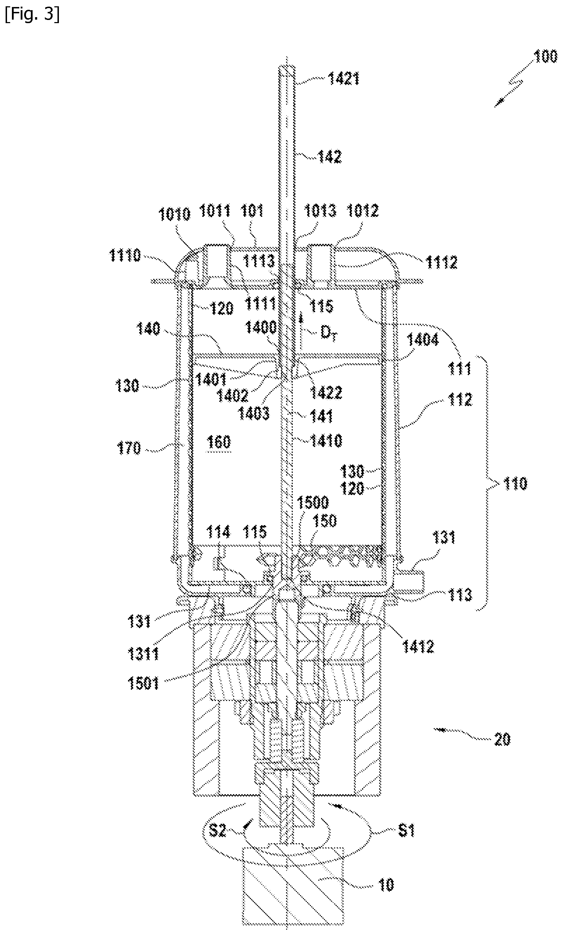

Selon un aspect particulier, la tige filetée 141 peut être logée dans une gaine de protection 142. La gaine de protection 142 qui s'étend entre une extrémité supérieure 1421 et une extrémité inférieure 1422 permet d'éviter à la tige filetée 141 d'entrer en contact avec le tissu adipeux qui en s'accumulant au niveau du taraudage 1403 du plateau de collecte 140 peut bloquer le déplacement de celui-ci. La figure 3 montre le dispositif de purification 100 après actionnement du moteur électrique 10 dans le deuxième sens de rotation S₂ permettant d'entraîner en rotation la tige filetée 141. La rotation de la tige filetée 141 entraîne la translation verticale du plateau de collecte 140 suivant la direction D_{T}.

L'extrémité inférieure 1422 de la gaine de protection est fixée dans l'ouverture 1400 du plateau de collecte 140. Afin de permettre le mouvement de la gaine de protection 142 lors du déplacement du plateau 140, le capot 111 et le couvercle 101 comportent respectivement une ouverture 1113 et une ouverture 1013 au travers desquelles la gaine 142 coulisse. Un joint 115 est présent autour de l'ouverture 1113 afin de préserver l'étanchéité en sommet de la chambre de centrifugation.

On note que, lorsque le dispositif de purification ne comprend pas de plateau de collecte mobile en translation, un embrayage bidirectionnel n'est pas nécessaire et le moteur électrique ou tout autre moyen d'entrainement en rotation peut être directement relié au plateau rotatif.

Dans un autre exemple de réalisation, le filtre est en un matériau rigide autoporteur. Le filtre peut être notamment réalisé à partir d'un feuillard métallique dans lequel des trous sont réalisés par laser, jet d'eau ou découpe chimique afin de former un crible, une grille métallique tissée de fils ou des billes agglomérées par frittage de poudres métalliques ou céramiques. Dans ce cas, l'élément de rigidification 130 n'est plus nécessaire et c'est le filtre autoporteur qui est directement en prise avec le plateau rotatif 131.

## Revendications

1. Dispositif pour la purification (100) d'un tissu adipeux comprenant au moins une enceinte étanche (110), un filtre (120) présent dans l'enceinte étanche, ledit filtre délimitant une chambre de centrifugation (160) pour un tissu adipeux et un moyen d'entrainement en rotation du filtre (10, 20), le filtre présentant une taille de pore configurée pour laisser passer un milieu liquide et retenir un tissu adipeux, **caractérisé en ce qu'**il comprend en outre un plateau de collecte (140) présent dans la chambre de centrifugation, le plateau de collecte étant mobile en translation suivant l'axe de rotation du filtre.

2. Dispositif selon la revendication 1, comprenant en outre une tige filetée (141) s'étendant dans la chambre de centrifugation (160) et coopérant avec une portion taraudée (1403) du plateau de collecte (140), la tige filetée étant reliée au moyen d'entrainement en rotation du filtre (10), ledit moyen étant configuré pour entraîner la tige filetée en rotation suivant un sens de rotation (S₂) opposé au sens de rotation du filtre (S₁).

3. Dispositif selon la revendication 2, comprenant en outre une gaine de protection (142) entourant la tige filetée (141).

4. Dispositif selon l'une quelconque des revendications 1 à 3, comprenant en outre un élément de rigidification (130) présent entre l'enceinte étanche (110) et le filtre (120).

5. Dispositif selon l'une quelconque des revendications 1 à 3, dans lequel le filtre est en un matériau rigide autoporteur.

6. Dispositif selon l'une quelconque des revendications 1 à 5, dans lequel le moyen d'entrainement en rotation du filtre est manuel.

7. Dispositif selon l'une quelconque des revendications 1 à 5, dans lequel le moyen d'entrainement en rotation du filtre comprend un moteur électrique (10).

## Patentansprüche

1. Vorrichtung zur Reinigung (100) eines Fettgewebes, umfassend mindestens eine dichte Umhüllung (110), einen Filter (120), der in der dichten Umhüllung vorhanden ist, wobei der Filter eine Zentrifugalkammer (160) für ein Fettgewebe und eine Einrichtung zum Drehantrieb des Filters (10, 20) begrenzt, der Filter eine Porengröße aufweist, die konfiguriert ist, um ein flüssiges Medium passieren zu lassen und ein Fettgewebe zurückzuhalten, **dadurch gekennzeichnet, dass** sie ferner eine Sammelplatte (140) umfasst, die in der Zentrifugalkammer vorhanden ist, wobei die Sammelplatte entlang der Drehachse des Filters translatorisch beweglich ist.

2. Vorrichtung nach Anspruch 1, ferner umfassend eine Gewindestange (141), die sich in die Zentrifugalkammer (160) erstreckt und mit einem Gewindeabschnitt (1403) der Sammelplatte (140) zusammenwirkt, wobei die Gewindestange mit der Einrichtung zum Drehantrieb des Filters (10) verbunden ist, wobei die Einrichtung konfiguriert ist, um die Gewindestange in einer Drehrichtung (S₂) entgegengesetzt zu der Drehrichtung des Filters (S₁) in Drehung zu versetzen.

3. Vorrichtung nach Anspruch 2, ferner umfassend eine Schutzhülle (142), die die Gewindestange (141) umgibt.

4. Vorrichtung nach einem der Ansprüche 1 bis 3, ferner umfassend ein Versteifungselement (130), das zwischen der dichten Umhüllung (110) und dem Filter (120) vorhanden ist.

5. Vorrichtung nach einem der Ansprüche 1 bis 3, wobei der Filter aus einem steifen, selbsttragenden Material ist.

6. Vorrichtung nach einem der Ansprüche 1 bis 5, bei dem die Einrichtung zum Drehantrieb des Filters manuell ist.

7. Vorrichtung nach einem der Ansprüche 1 bis 5, wobei die Einrichtung zum Drehantrieb des Filters einen Elektromotor (10) umfasst.

## Claims

1. A device (100) for purifying adipose tissue, comprising at least one sealed enclosure (110), a filter (120) present in the sealed enclosure, said filter delimiting a centrifugation chamber (160) for centrifuging adipose tissue, and a means (10, 20) for rotating the filter, the filter having a pore size configured to allow a liquid medium to pass and to retain adipose tissue, **characterised in that** it further comprises a collector plate (140) present in the centrifugation chamber, the collector plate being able to move in translation along the axis of rotation of the filter.

2. The device according to claim 1, further comprising a threaded rod (141) extending in the centrifugation chamber (160) and cooperating with a tapped portion (1403) of the collector plate (140), the threaded rod being connected to rotary drive means of the filter (10), said means being configured to drive the threaded rod in rotation, in a direction of rotation (S₂) opposite to the direction of rotation (S₁) of the filter.

3. The device according to claim 2, further comprising a protective sleeve (142) surrounding the threaded rod (141).

4. The device according to any one of claims 1 to 3, further comprising a stiffening element (130) present between the sealed enclosure (110) and the filter (120).

5. The device according to any one of claims 1 to 3, wherein the filter is made of a rigid self-supporting material.

6. The device according to any one of claims 1 to 5, wherein the rotary drive means of the filter is manual.

7. The device according to any one of claims 1 to 5, wherein the rotary drive means of the filter comprises an electric motor (10).
